# EUROPEAN PATENT SPECIFICATION

(11) **EP 3 080 145 B1**
(45) Date of publication and mention of the grant of the patent: **17.07.2019**
(21) Application number: 14821228.5
(22) Date of filing: 03.12.2014
(51) Int. Cl.: C07K 14/37, A23L 29/00, A61K 47/00

(54) **A METHOD FOR INCREASING PRODUCT STABILITY WITH HYDROPHOBIN VARIANTS**
VERFAHREN ZUR ERHÖHUNG DER STABILITÄT VON PRODUKTEN DURCH HYDROPHOBIN-VARIANTEN
MÉTHODE POUR AUGMENTER LA STABILITÉ DE PRODUITS AVEC DES VARIANTS D'HYDROPHOBINE

(30) Priority: 03.12.2013 FI 20136214
(43) Date of publication of application: 19.10.2016
(73) Proprietor: Teknologian Tutkimuskeskus VTT OY, 02150 Espoo (FI)
(72) Inventor: PAANANEN, Arja, 02044 VTT (FI); LIENEMANN, Michael, 02044 VTT (FI); LINDER, Markus, 00200 Helsinki (FI)
(74) Representative: Boco IP Oy Ab
(86) International application number: PCT/FI2014/050952
(87) International publication number: WO 2015/082772

(56) References cited:
- M. LIENEMANN ET AL: "Structure-Function Relationships in Hydrophobins: Probing the Role of Charged Side Chains", APPLIED AND ENVIRONMENTAL MICROBIOLOGY, vol. 79, no. 18, 8 July 2013 (2013-07-08), pages 5533-5538, XP055173365, ISSN: 0099-2240, DOI: 10.1128/AEM.01493-13
- RYOTA YAMASAKI ET AL: "Electrochemical properties of honeycomb-like structured HFBI self-organized membranes on HOPG electrodes", COLLOIDS AND SURFACES B: BIOINTERFACES, vol. 123, 1 November 2014 (2014-11-01), pages 803-808, XP055173644, ISSN: 0927-7765, DOI: 10.1016/j.colsurfb.2014.10.018
- COX A R ET AL: "Exceptional stability of food foams using class II hydrophobin HFBII", FOOD HYDROCOLLOIDS, ELSEVIER BV, NL, vol. 23, no. 2, 1 March 2009 (2009-03-01), pages 366-376, XP026128626, ISSN: 0268-005X, DOI: 10.1016/J.FOODHYD.2008.03.001 [retrieved on 2008-03-07]
- COX R A ET AL: "Surface properties of class II hydrophobins from Trichoderma reesei and influence on bubble stability", LANGMUIR, AMERICAN CHEMICAL SOCIETY, NEW YORK, NY; US, vol. 23, no. 15, 17 July 2007 (2007-07-17) , pages 7995-8002, XP002548622, ISSN: 0743-7463, DOI: 10.1021/LA700451G [retrieved on 2007-06-20]
- ZEFANG WANG ET AL: "Mechanisms of Protein Adhesion on Surface Films of Hydrophobin", LANGMUIR, vol. 26, no. 11, 1 June 2010 (2010-06-01), pages 8491-8496, XP055173842, ISSN: 0743-7463, DOI: 10.1021/la101240e
- LINDER ET AL: "Hydrophobins: Proteins that self assemble at interfaces", CURRENT OPINION IN COLLOID AND INTERFACE SCIENCE, LONDON, GB, vol. 14, no. 5, 1 October 2009 (2009-10-01), pages 356-363, XP026498520, ISSN: 1359-0294, DOI: 10.1016/J.COCIS.2009.04.001 [retrieved on 2009-05-08]

## Description

### FIELD OF THE INVENTION

The present invention relates to compositions comprising hydrophobin variants, optionally containing a small fusion partner. Furthermore, the invention relates to methods and uses for increasing product stability with hydrophobin variants. In particular the present invention relates to hydrophobins as stabilizers in foams and emulsions.

### BACKGROUND OF THE INVENTION

Foam stability is important in various fields of technology. Enhancement of foam stability by hydrophobins has been reported e.g. for food applications [1].

Hydrophobins are adhesive proteins produced by filamentous fungi. They are important during various growth and development stages during which they, for example, form protective spore coatings [2, 3] and constituents of the hyphal cell wall [4]. Furthermore, hydrophobins support fungal growth by lowering the water surface tension for hyphal penetration of the air-water interface [5, 6]. The latter process involves self-assembly of spread out hydrophobin monolayers at the air-water interface [7, 8] which may be recognized by other proteins at the surface of the fungal cell. Recently the structure-function relationships of Class II hydrophobin HFBI has been investigated by means of site-directed mutagenesis to gain an understanding of the role of charged amino acids in the structure of hydrophobins [13].

WO 2010/060811 discloses use of hydrophobin in galenic applications in pharmaceutical technology. US 2013/0303631 discloses use of hydrophobin in topical compositions. Lienemann et al. 2013 [19] disclose a hydrophobin fusion protein GFP-HFBI, in which the fusion partner GFP, however, has a disadvantage of having a very large size. Yamasaki et al. 2014 [23] disclose that wild-type hydrophobin HFBI and point-mutated variants of HFBI all form self-organized membranes that have the same typical structure. These membranes appeared to have a periodic and densely packed structures. However, this work is about hydrophobin layers on solid support, not at the air-water interface.

### SUMMARY OF THE INVENTION

An object of the present disclosure is to provide a hydrophobin composition comprising a variant of class II hydrophobin, especially a composition comprising *Trichoderma reesei* hydrophobin HFBI, which is useful for formation of strong protein films at the air-water interface or oil-water interface. The composition optionally contains a small fusion partner, which is of equal or smaller size.

The present invention discloses a hydrophobin variant of class II hydrophobin comprising an amino acid sequence having a substitution of one or more charged amino acid residues against sterically related non-charged homologues in amino acid sequence of the mature hydrophobin HFBI of SEQ ID NO:2.

The present disclosure relates to a composition comprising a hydrophobin variant of class II hydrophobin characterized in that the variant comprises an amino acid sequence having at least 80%, preferably at least 95% identity to the amino acid sequence of SEQ ID NO:2 and the variant comprises charged residues, wherein the variant has surface activity and capability to form strong interfacial films.

An embodiment of the disclosure relates to a variant comprising at least 80%, preferably at least 81%, 82%, 83%, 84%, 85%, 86%, 87%, 88%, 89%, 90%, 91%, 92%, 93%, or 94% identity, most preferably at least 95%, 96%, 97%, 98%, 99%, or 100% identity to the amino acid sequence of the mature hydrophobin HFBI of SEQ ID NO:2.

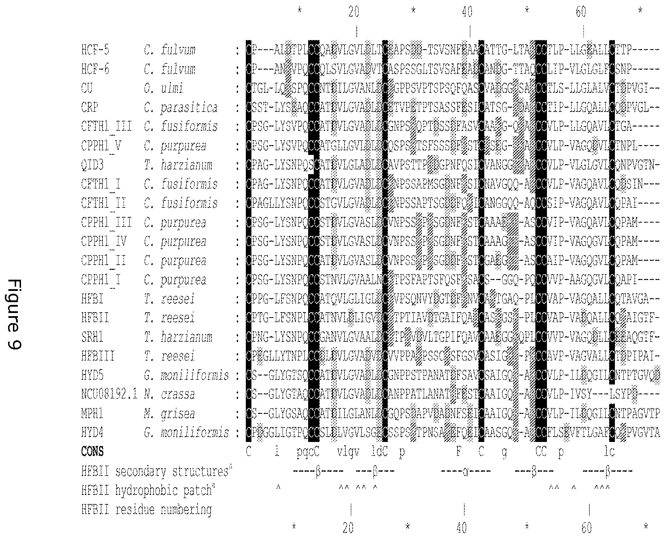

In a preferred embodiment the present disclosure relates to class II hydrophobins which have been modified by any of the following amino acid substitutions:
(i) substitution of a basic amino acid between the conserved cysteine #4 (Position 25 in sequence alignment of Figure 9) and #5 (Position 42 in sequence alignment of Figure 9) against a non-charged homologue;
(ii) substitution of a basic amino acid between the conserved cysteine #5 (Position 42 in sequence alignment of Figure 9) and #6 (Position 52 in sequence alignment of Figure 9) against a non-charged homologue;
(iii) substitution of an acidic amino acid between the conserved cysteine #3 (Position 13 in sequence alignment of Figure 9) and #5 (Position 42 in sequence alignment of Figure 9) against a non-charged homologue;

In a preferred embodiment the present invention relates to a variant comprising as a fusion partner a fusion protein of equal or smaller size than said hydrophobin variant.

In an embodiment of the disclosure the composition of the present invention may further comprise a peptide, which has equal or smaller dimensions than the variant of the invention. Further included are the aforementioned peptides to which organic or inorganic molecules have been linked through covalent bonds. Examples of such modifications are covalent bonding by conjugation of cysteine and maleimide residues and conjugation of carboxyl and amino groups through carbodiimide crosslinkers such as 1-Ethyl-3-(3-dimethylaminopropyl)carbodiimide.

The interfacial film formed by the polypeptide variants of the present disclosure is very strong and this property results in improved stability of foams and emulsions by making air bubbles or oil droplets stronger against coalescence and disproportionation (Ostwald ripening).

A variant or the present invention may comprise *Trichoderma reesei* hydrophobin HFBI. There are several Class II hydrophobins originating from filamentous fungi e.g. *Trichoderma reesei,* which are suitable in the present disclosure.

According to a preferred embodiment of the disclosure the variant comprises an amino acid sequence having a substitution of one or more amino acid residues at positions 30, 32, 40, 43, 45 or 50 of the hydrophobin, wherein the amino acid positions correspond to the amino acid sequence of the mature hydrophobin HFBI of SEQ ID NO:2.

According to a preferred embodiment of the disclosure the composition and the variant of the present invention provide improved colloidal stability to foams and emulsions compared to the wild type mature hydrophobin HFBI of sequence SEQ ID NO:2.

According to a preferred embodiment of the disclosure the composition and the variant of the present invention provide improved formation of an elastic protein film compared to the wild type mature hydrophobin HFBI of sequence SEQ ID NO:2.

According to a preferred embodiment of the disclosure the variant comprises an amino acid sequence set forth in SEQ ID NO:2 with one or more substitutions selected from the group consisting of D30N, K32Q, D40Q, D43N, R45Q, and K50Q, wherein the positions of the amino acid substitutions are numbered in reference to the positions in SEQ ID NO:2. The variant may be selected from the group consisting of SEQ ID NO:7, SEQ ID NO:9 and SEQ ID NO:10.

The present invention relates to a method for increasing product stability in products containing air-water or oil-water interface, characterized in that the method comprises the step of using a hydrophobin composition according to the present invention, which composition has been defined above.

According to an embodiment of the method of the present invention product stability is increased by forming a strong and stable foam or emulsion, a strong elastic film, or providing improved visco-elastic properties of interfacial films.

In an embodiment of the invention is provided a method for increasing product stability at air-water interface or oil-water interface of a foam or emulsion, characterized in that the method comprises the step of using the hydrophobin composition of the present invention.

Product stability of foams and emulsions can be increased by improving properties of either the continuous phase or the air-water or oil-water interface, or both continuous phase and interface. By strengthening the visco-elastic properties of the interface, more elastic and stronger interfacial films are formed and the bubbles and oil droplets are more resistant to coalescence and disproportionation.

Instead of air any inert gas, or a mixture of air and any inert gas is suitable in the present disclosure.

According to an embodiment of the present invention the product containing air-water or oil-water interface is selected from the group consisting of food products, personal care products, paints, pharmaceuticals, and drugs.

The present invention relates to use of the hydrophobin composition according to the present invention as a stabilizer, preferably as a stabilizer in foams or emulsions. In other words, a composition comprising a hydrophobin variant of class II hydrophobin, wherein the variant comprises an amino acid sequence having at least 80%, preferably at least 95% identity to the amino acid sequence of SEQ ID NO:2 and the variant comprises charged residues, wherein the variant has surface activity and capability to form strong interfacial films, is useful in food products, personal care products, paints, pharmaceuticals, and drugs.

The present invention also relates to use of the hydrophobin composition according to the present invention in personal care products or in paints.

A hydrophobin composition according to the present disclosure can be used in a medicament. A hydrophobin composition according to the present invention can be used in a food product.

### BRIEF DESCRIPTION OF THE DRAWINGS

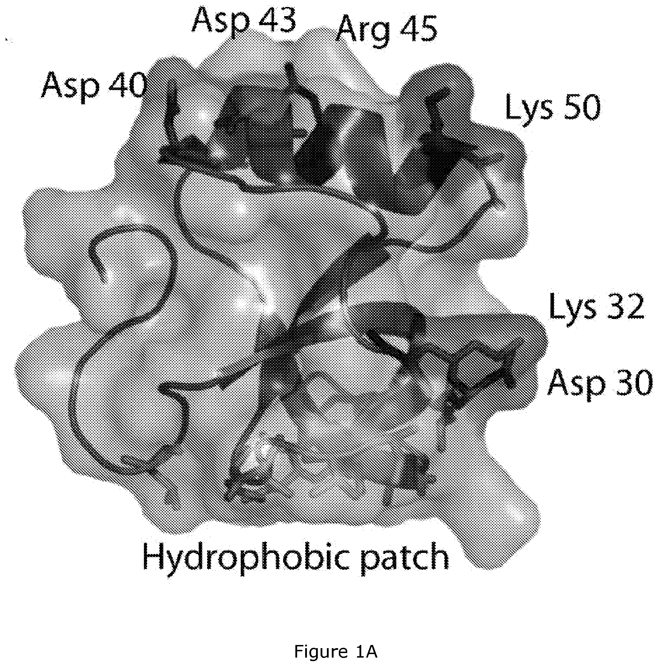

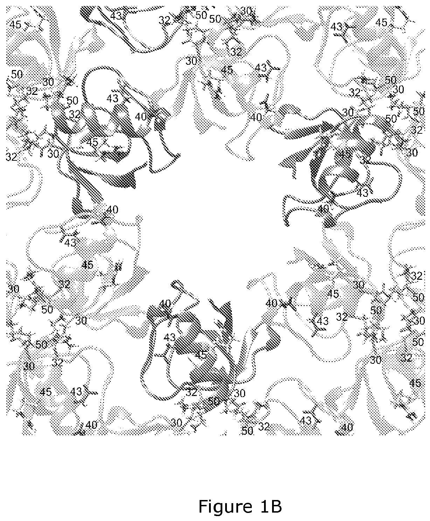

**Figure 1****: A)** Three-dimensional structure of *Trichoderma reesei* hydrophobin HFBI (PDB-ID 2FZ6). The hydrophobic patch provides attractive interaction during adsorption to hydrophobic substrates and to the gaseous phase during the formation of monolayers at the air-water interface. **B)** Computational model of HFBI monolayer with 6-fold symmetry (referred to as β structure by Magarkar et al. [21]). The side chain atoms of the mutated residues are shown as sticks and the positions of the mutated side residues in the HFBI sequence are indicated next to the corresponding alpha-carbon atom. Individual HFBI molecules of the pore forming hexamer complex are highlighted by shading. Pores in the layer are evident that are flanked by the negatively charged side chains of residues D40 and D43 as well as the positively charged side chain of R45.

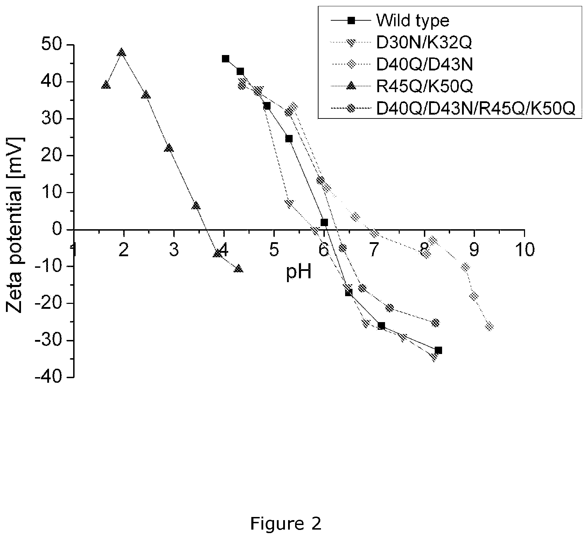

**Figure 2****:** Zeta potential of HFBI wild type and variants D30N/K32Q (SEQ ID NO:7), D40Q/D43N (SEQ ID NO:8), D40Q/D43N/R45Q/K50Q (SEQ ID NO:9) and R45Q/K50Q (SEQ ID NO:10) measured in 0.1 mg/ml protein solutions. The isoelectric points were determined from this plot as 6.1, 5.8, 7.0, 6.2 and 3.6, respectively.

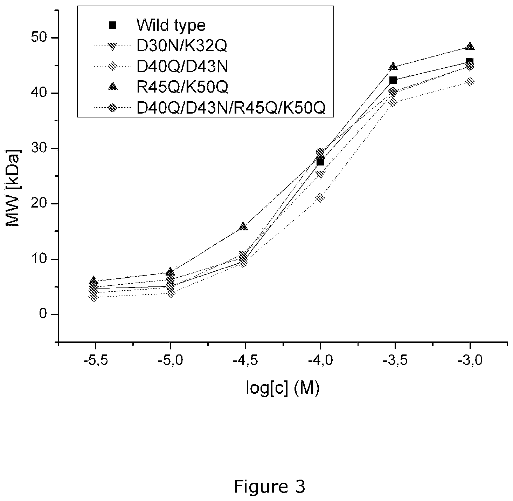

**Figure 3****:** Concentration dependence of HFBI variant aggregation. The molecular weight of homodimers is plotted against the HFBI variant concentration.

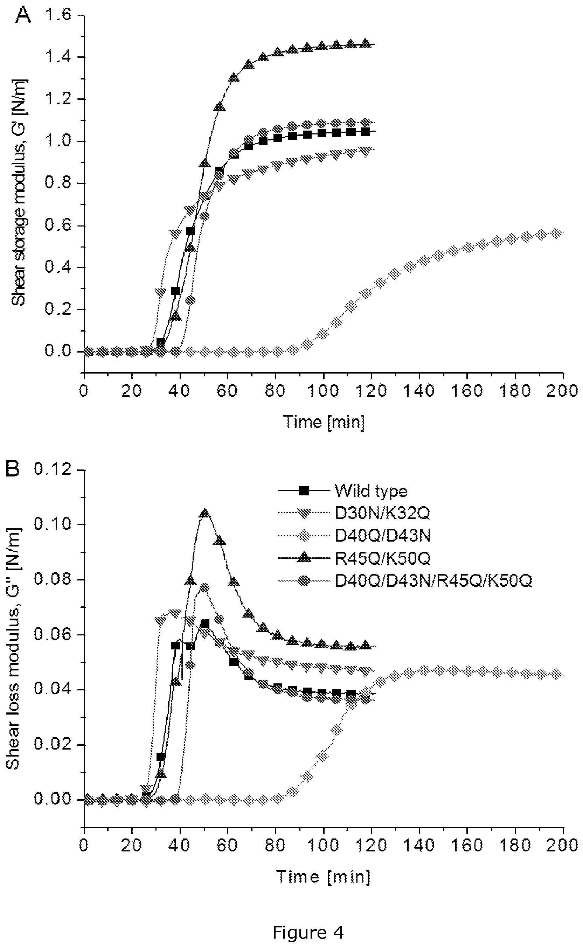

**Figure 4****:** Interfacial rheology data (storage modulus, G' = A; loss modulus, G" = B) of HFBI wild type and HFBI variants D30N/K32Q, D40Q/D43N, R45Q/K50Q and D40Q/D43N/R45Q/K50Q at the air-water interface as a function of adsorption time. The interfacial layers are adsorbed from 0.3 µM protein solutions.

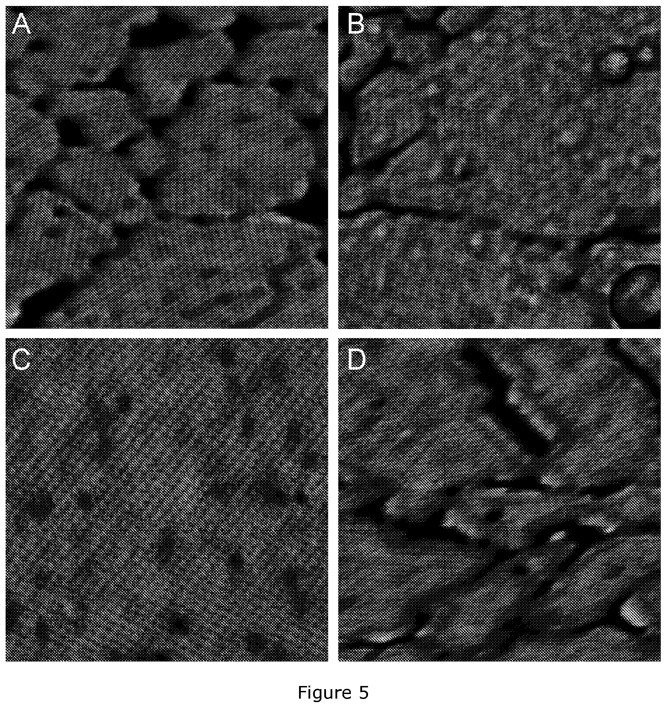

**Figure 5****:** AFM images of LB-films of HFBI that were assembled on the air-water interface and have been transferred to a flat mica substrate, dried and imaged in air using tapping mode. Displayed are typical 200 nm phase images of the HFBI variants D30N/K32Q, D40Q/D43N, R45Q/K50Q and D40Q/D43N/R45Q/K50Q (A-D, respectively).

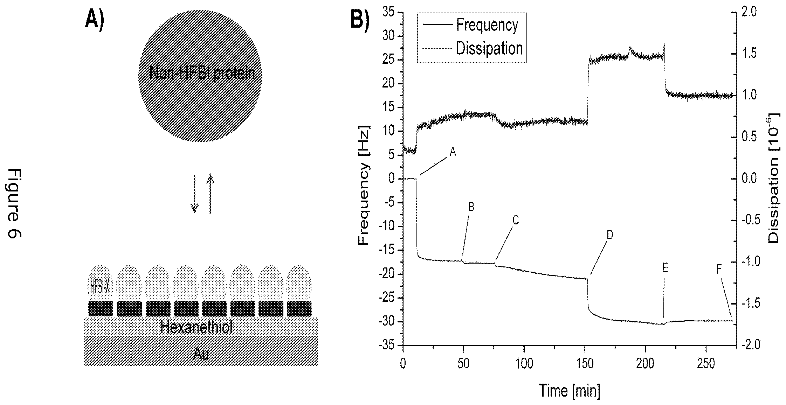

**Figure 6****: A)** Schematic representation of the HFBI coated QCM sensor at which adsorption of non-HFBI proteins was measured; **B)** Representative protein adsorption QCM experiment showing the change of frequency (bottom graph) and dissipation signal (top graph) during sequential adsorption of HFBI variant D40Q/D43N at pH 9.0 and glucose oxidase. The marked onsets of experimental phases are: (A) Injection of 0.03 mg HFBI-D40Q/D43N in 10 mM Na acetate buffer (pH 5.5), (B) removal of unbound hydrophobin by buffer rinsing, (C) equilibration with 10 mM glycine buffer (pH 9.0), (D) injection of 0.3 mg glucose oxidase at pH 9.0, (E) washing off of unbound glucose oxidase, (F) end of experiment. The adsorbed mass of the non-HFBI protein was calculated using the frequencies at points D and F.

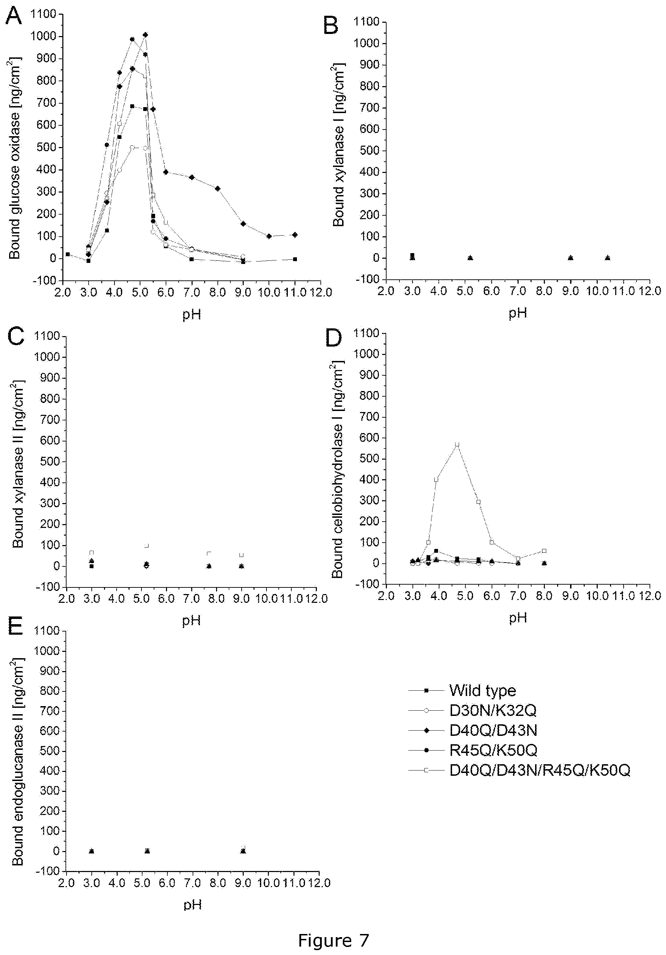

**Figure 7****:** pH-Dependent adsorption of non-HFBI proteins to adsorbed layers of HFBI wild type and HFBI variants D30N/K32Q, D40Q/D34N, D40Q/D34N/R45Q/K50Q and R45Q/K50Q determined by QCM-D. The injected non-HFBI proteins were *Aspergillus niger* glucose oxidase (**A**, pI 4.2) and the *Trichoderma reesei* proteins xylanase I (**B**, pI 9), xylanase II (**C**, pI 5.5), cellobiohydrolase I (**D**, pI 3.7-4.2) and endoglucanase II (**E**, pI 5.2). The adsorption data on glucose oxidase binding to HFBI wild type layers was originally published in reference [9].

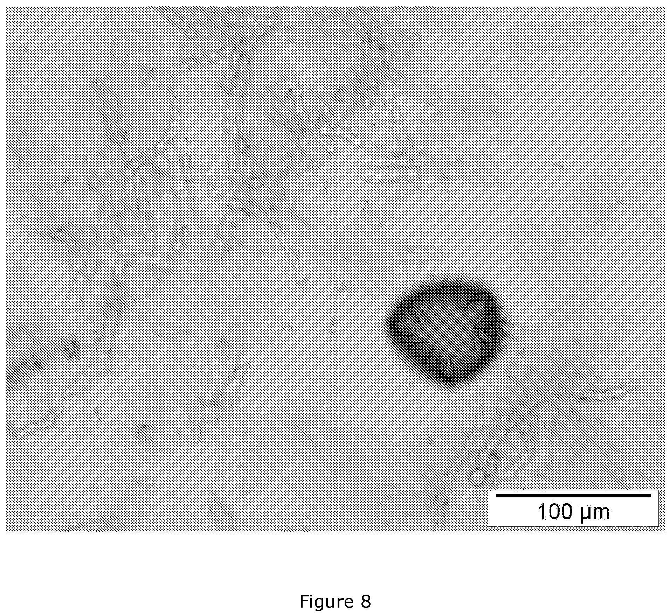

**Figure 8****:** Culture supernatant of *T*. *reesei* strain M411 secreting HFBI variant D30N/K32Q. The wrinkles of the air bubble next to fungal mycelium indicate the presence of hydrophobins that assemble as monolayers at the air-water interface.
**Figure 9****:** Sequence alignment from deduced Class II hydrophobin protein sequences available from databases during the year 2004 prepared with Clustal W [10]. TH HCF-5 of *Cladosporium fulvum* (accession AJ133703), HCF-6 of *Cladosporium fulvum* (AJ251294), CU of *Ophiostoma ulmi* (U00963), CRP of *Cryphonectria parasitica* (L09559), CFTH1_I-III of *Claviceps fusiformis* (AJ133774), CPPH1_I-V of *Claviceps purpurea* (AJ418045), QID3 of *Trichoderma harzianum* (X71913), HFBI of *Trichoderma reesei* (Z68124), HFBII of *Trichoderma reesei* (Y11894), SRH1 of *Trichoderma harzianum* (Y11841), HFBIII of *Trichoderma reesei* (published in [12]), HYD5 of *Gibberella moniliformis* (AY158024), NCU08192.1 of *Neurospora crassa* (AABX01000408), MHP1 of *Magnaporthe grisea* (AF126872), HYD4 of *Gibberella moniliformis* (AY155499) were aligned using the Gonnet matrix series [13]. The shown sequences were obtained by performing a search in protein databases for annotated hydrophobin sequences showing the typical Cys patterning where protein fragments were excluded. The amino termini sequences were found to be highly variable and only sequences after the first Cys were used in the alignments for clarity. The Cys-pattern is shown by alignment of the Cys residues (marked with black background shading). The displayed HFBII sequence begins at position three. ^{a}Annotations of the secondary structure elements and the residues forming the hydrophobic patch are given according to the published HFBII crystal structure [14]. Conserved residues in the consensus sequences are shown in lower case (80%) and upper case (100%). The figure was modified from [11] with acidic residues Asp (pKa = 3.71) and Glu (pKa = 4.15) marked with light grey background shading and basic residues His (pKa = 6.04), Lys (pKa = 10.67) and Arg (pKa = 12.10) with dark background shading.

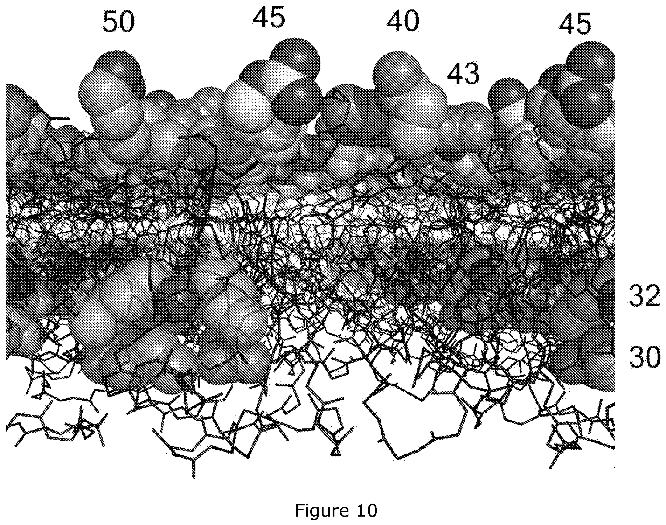

**Figure 10****:** Side view of the modelled HFBI monolayer shown in Figure 1B. The location of residues D30 and K32 inside the layer close to the hydrophobic patch (bottom) is evident.

### SEQUENCE LISTING

**SEQ ID NO:1** The amino acid sequence of the proenzyme form of *T. reesei* HFBI hydrophobin. The signal and propeptide are displayed in bold and italics, respectively (removed during secretion). HFBI residues D30, K32, D40, D43, R45 and K50 that were substituted in the corresponding variants are underlined.
**SEQ ID NO:2** The amino acid sequence of the mature form of *T. reesei* HFBI hydrophobin.
**SEQ ID NO:3** Sequence of a probe complementary to *hph* resistance marker. The probe was used in PCR for confirming the *hfb2* gene disruption.
   ATTCTTCGCCCTCCGAGAGC
**SEQ ID NO:4** Sequence of a probe complementary to the *hfb2* region located upstream of the deletion cassette insertion. The probe was used in PCR for confirming the *hfb2* gene disruption. GAATCATGCTGGAGTGAAGG
**SEQ ID NO:5** Sequence of a primer that binds to the CBHI promoter. The primer was used for analysing acetamide-resistant clones for HFBI variant gene insertion by sequencing. CAACTCAGATCCTCCAGGAGAC
**SEQ ID NO:6** Sequence of a primer that binds to the CBHI terminator. The primer was used for analysing acetamide-resistant clones for HFBI variant gene insertion by sequencing. TCATGATACGGGCTCACCAAG
**SEQ ID NO:7** The amino acid sequence of a variant D30N/K32Q.
**SEQ ID NO:8** The amino acid sequence of a variant D40Q/D43N.
**SEQ ID NO:9** The amino acid sequence of a variant D40Q/D43N/R45Q/K50Q.
**SEQ ID NO:10** The amino acid sequence of a variant R45Q/K50Q.

### DEFINITIONS

The term **"water"** refers to tap water, deionized or distilled water that may contain water soluble chemical substances.

The term **"oil"** refers to any nonpolar chemical substance, animal, vegetable or petrochemical in origin, that is immiscible with water and is viscous liquid at ambient temperatures.

The phrase **"a polypeptide variant has surface activity"** means that the molecule is amphiphilic containing both hydrophilic and hydrophobic groups and hence has tendency to accumulate at the interface and lower the surface (or interfacial) tension between air and water, or between oil and water.

The term **"strong interfacial film"** refers to fluid interface between air and water, or oil and water possessing surface elastic modulus over 0.1 N/m. (Value as presented by [1].) The value is derived from the theoretical calculations by Kloek et al [22].

The phrase **"improved formation of an elastic protein film"** means that formed interfacial film has improved visco-elastic properties showing increased surface elastic modulus values.

### DETAILED DESCRIPTION OF THE INVENTION

The present invention relates to a composition comprising variant of class II hydrophobins. Especially the invention relates to improving interfacial film properties.

In a specific embodiment the present disclosure relates to the self-assembly processes and interfacial rheology properties of wild type HFBI and HFBI variants. The results give new insight into the structure-function relationship.

The present disclosure relates to a mutated surface-active protein that is capable of forming very strong elastic films. Particularly, the present disclosure relates to hydrophobin (HFBI) variant produced by means of molecular biology. Especially the disclosure relates to charge-based engineering of *Trichoderma reesei* hydrophobin HFBI. Hydrophobin has effect on the interfacial assembly and interaction with soluble proteins.

Hydrophobins have many roles in fungal development such as lowering the surface tension of surrounding water for hyphal penetration, adhesion to surfaces, and making coatings. These proteins have a unique amphiphilic structure. They tend to associate with each other and assemble at the air-water interface into highly ordered monolayers that are strong and elastic [7].

Hydrophobins are amphiphilic proteins that are secreted by filamentous fungi for hyphal penetration of the air-water interface and form a protective coating on the outside of fungal spores. These proteins have the tendency to associate with each other and attach as coating to hydrophobic substrates and form highly ordered monolayers at the air-water interface. These properties allow protein-based surface functionalization. A detailed knowledge of the molecular basis of hydrophobin substrate interaction is highly desirable for the design of fusion proteins and improve the understanding of self-assembly processes.

The function of charged amino acids of HFBI i.e. D40, D43, R45 and K50 is probed at the solvent-exposed hydrophobin face and D30, K32 close to the hydrophobic patch. The oligomerisation and layer formation appear to be largely unaffected by the tested charge variations. However, the lateral interactions between hydrophobin molecules that are important for the viscoelastic properties of the interfacial layer are affected by the mutations. The interfacial rheology data shows clear differences between hydrophobin variants, even higher storage modulus values than for the wild type HFBI. Addition of fungal carbohydrate active enzymes to assembled hydrophobin variant monolayers suggests a strong role of the resulting charge pattern in secondary protein adhesion.

Recent studies have shown secondary protein adhesion at hydrophobin layers on a solid substrate [9]. Understanding the molecular basis for secondary protein adhesion and hydrophobin-hydrophobin interaction contributes valuable insights for the field of protein self-assembly and may allow the engineering of protein layers for specific ligand binding. A recently published structural model of the *Trichoderma* reesei hydrophobin HFBI (7.5 kDa) has shown that these layers expose ionizable groups (Figure 1) which may be recognized by other proteins.

The present disclosure identifies which charged HFBI amino acid residues are responsible for the adhesion of proteins to HFBI monolayers. For this purpose, four HFBI variants with altered charges were produced and characterized with respect to their isoelectric point, aggregation and monolayer formation at the air-water interface as well at a positively charged solid substrate.

According to the present disclosure the variant can comprise an amino acid sequence inside a protein region that is homologous to the alpha helix of HFBI (See Figure 9) with substitutions of one or more basic amino acids (e.g. Arg, His, Lys) against non-charged homologous residues.

According to the present disclosure any substitution of a charged amino acid of any class II hydrophobin can be used for improving the stability of interfacial films. Increasing product stability means that the structure of foams or emulsions is more stable because they have stronger interfaces. The strength is described by the visco-elastic properties. Increased product stability improves for example the shelf-life of the products.

Furthermore, improvement of hydrophobin production yield (e.g. concentration of hydrophobin available) in the supernatant by substitutions of any charged residues in any class II hydrophobin is obtained.

The variant D30N/K32Q (SEQ ID NO:7) has a substitution of aspartic acid to asparagine at position 30 and a substitution of lysine to glutamine at position 32 of the mature hydrophobin HFBI (SEQ ID NO:2).

The variant D40Q/D43N (SEQ ID NO:8) has a substitution of aspartic acid to glutamine at position 40 and a substitution of aspartic acid to asparagine at position 43 of the mature hydrophobin HFBI (SEQ ID NO:2).

The variant D40Q/D43N/R45Q/K50Q (SEQ ID NO:9) has a substitution of aspartic acid to glutamine at position 40, a substitution of aspartic acid to asparagine at position 43, a substitution of arginine to glutamine at position 45 and a substitution of lysine to glutamine at position 50 of the mature hydrophobin HFBI (SEQ ID NO:2).

The variant R45Q/K50Q (SEQ ID NO:10) has a substitution of arginine to glutamine at position 45 and a substitution of lysine to glutamine at position 50 of the mature hydrophobin HFBI (SEQ ID NO:2).

Finally, the importance of the HFBI wild type and variant layer charges for secondary protein adhesion was studied by monitoring the adhesion of other *Trichoderma reesei* proteins and glucose oxidase at different pH's. The given experiments showed that the introduced mutations did not disturb the HFBI layer assembly but could confer selective secondary protein binding as shown for the *T. reesei* cellobiohydrolase CBHI and the HFBI variant D40Q/D43N.

The high surface elasticity relates to product stability. The surface shear rheology value (storage modulus) at the air-water interface reported for equilibrated film adsorbed from 1 µM solution of HFBII and HFBI is appr. 0.5 N/m and appr. 0.7 N/m, respectively (Cox et al 2007 [15]). At similar measurement conditions, but with less protein (0.3 µM) we have measured a corresponding value for HFBI to be appr. 1.0 N/m, and for the new HFBI-variant appr. 1.4 N/m, which is to inventors' knowledge the highest value ever measured for proteins. Beneficial in this disclosure is that this new protein variant enables formation of stronger and more stable foams with less consumption of protein. In addition, production of the variant of the present disclosure is easier than for the wild type protein and can hence be produced in higher amounts.

The composition according to the present invention comprises a hydrophobin variant of class II hydrophobin, wherein said variant comprises an amino acid sequence having a substitution of one or more charged amino acid residues against sterically related non-charged homologues in amino acid sequence of the mature hydrophobin HFBI of SEQ ID NO:2. The variant of the present disclosure has improved colloidal stability to foams and emulsions or improved formation of an elastic protein film compared to the wild type mature hydrophobin HFBI of sequence SEQ ID NO:2.

In the present disclosure mutations made to a hydrophobin molecule by molecular biology means enhances the formation of a highly elastic protein film at the air-water interface. The high surface elasticity relates to foam stability. The mutations made are shown in Lienemann et al. 2013 [19] for a HFBI-GFP fusion protein.

The composition of the present disclosure comprises a variant of class II hydrophobin, wherein the variant comprises an amino acid sequence having at least 80%, preferably 95% identity to the amino acid sequence of SEQ ID NO:2 and the variant comprises charged residues, wherein the variant has surface activity and capability to form strong interfacial films.

According to an embodiment of the invention the composition may comprise a fusion protein with a fusion partner of equal or smaller size than said hydrophobin variant. In other words the non-hydrophobin part, is of equal or smaller size to hydrophobin variant. This novel fusion protein has advantages compared to a GFP-HFBI fusion protein.

The fusion partner of the present disclosure is special and distinct from GFP because it allows the hydrophobin parts to form the tight layers. The GFP fusion protein is much larger (mol. weight about 30 kDa) than HFBI (mol. weight about 7.5 kDa) and the size difference between these two parts of the fusion protein is therefore expected to prevent the ordered HFBI layer formation through steric restrictions. GFP is too bulky to fit into an HFBI layer at the HFBI-given density. A large fusion partner such as GFP prevents the formation of a strong interface. High surface elasticity relates to product stability.

In the present invention interfacial layer stabilization properties are improved.

The variants used in the present disclosure contain no fusion partner. The film strengths for all variants were measured by interfacial shear rheology technique. The surface shear rheology value (storage modulus) at the air-water interface for one of the variants (HFBI-R45Q/K50Q) was exceptionally high, being appr. 1.4 N/m for 0.3 µM solution. This is the highest value ever measured for proteins, and is 40% better value than for wild-type HFBI and twice as good as HFBII used in applications by Unilever [15].

The present disclosure provides improvement of existing technology for formation of strong protein films at the air-water interface. The interfacial rheology values for describing improved strength, and as a consequence improved stability, are appr. 40% higher for the HFBI variant than for the wild type HFBI. In addition, mutations promote secretion of HFBI-variants to supernatant (wild-type HFBI in mycelium), which facilitates purification of proteins remarkably. The yields for the produced variant protein are very good.

The present disclosure provides mutations made to the wild-type hydrophobin protein where charged amino acids have been replaced by uncharged residues. These mutations are known for GFB-HFBI fusion proteins.

The present invention provides hydrophobin compositions comprising different amino acid substitutions e.g. R45Q/K50Q, which contains a substitution of basic amino acids against non-charged sterically similar amino acids.

Substitutions D30N/K32Q, R45Q/K50Q and D40Q/D43N/R45Q/K50Q provide means for obtaining increased protein yields from the culture supernatant.

Substitution R45Q/K50Q provides a significant improvement in interfacial layer strength.

The present inventors show that the structure of HFBI monolayers appears to be largely unaffected by modification of charged HFBI residues but secondary protein binding to the HFBI layers can be adjusted by substitution of the acidic surface-exposed residues. These findings are useful for technical applications (such as creating HFBI-based pH-sensitive functional surface modifications) and a useful basis for further studies that elucidate the mechanism and natural significance of enzyme binding to hydrophobin-coated surfaces.

The technical core feature of the present disclosure is the use of the variants as stabilizers in different applications utilizing colloidal foams and emulsions. Colloidal stability is involved in wide variety of technologies such as personal care, cleaning, lubrication, paints, food technology, pharmaceuticals, drug delivery etc. Strong elastic protein films offer sustainable approach for stabilizing colloids, which in this particular example is colloidal foams. The primary application area is foams, where the interfacial elasticity determines the foam stability. The idea can be well applicable to emulsions as well. The potential application areas can be food technology, personal care, paints, pharmaceuticals, drug delivery.

Applications of the present disclosure include food foams that are based on the high surface elasticity of the protein films.

In food technology the products wherein the present disclosure is useful may be whipped cream, non-dairy cream, a frozen aerated confection, a mousse, mayonnaise, dressing, spread, soup, sauce or beverage.

### EXAMPLE

### Materials and Methods

### Reagents and Chemicals

HFBI variants were produced in *T. reesei* and purified from culture supernatant by reversed-phase chromatography as described below. Preparations of the *T. reesei* enzymes endoglucanase EGII, xylanases XYLI and XYLII and cellbiohydrolase CBHI were a courtesy of Matti Siika-Aho (VTT, Finland). Glucose oxidase from *Aspergillus niger* (GOx) and 1-hexanethiol were obtained from Sigma-Aldrich Chemie GmbH (Germany). The plasmid pTNS27 was provided by T. Nakari-Setälä (VTT, Finland; [4]). The *T*. *reesei* protein production strain M219 was obtained from M. Saloheimo (VTT, Finland).

### Production of HFBI variants in Trichoderma reesei

HFBI variants were produced in the genetically modified *Trichoderma reesei* strains VTT D-121448 (-D40Q/D43N/R45Q/K50Q), VTT D-121449 (-D40Q/D43N), VTT D-121450 (-R45Q/K50Q) and VTT D-121451. The production was performed in 1 | Trichoderma minimal medium (TrMM), which had the following composition: 0.11 M KH₂PO₄, 38 mM (NH₄)₂SO₄, 2.4 mM MgSO₄, 4.1 mM CaCl₂, 0.18 mM FeSO₄ × 7 H₂O, 95 µM MnSO₄ × H₂O, 49 µM ZnSO₄ × H₂O, 0.16 mM CoCl₂ × 6 H₂O and 2% (w/v) glucose, pH 5.2) supplemented with 4% (w/v) lactose and 2% (w/v) spent grain extract. The fungal cultivation was performed in shake flasks during seven days at 28°C with 200 rpm shaking after which the culture liquid was stored frozen until further processing.

### Purification of HFBI variants from Trichoderma reesei culture supernatant

Purification of the HFBI variants was performed using successive purification in an aqueous two-phase system (ATPS) and by reversed-phase chromatography essentially as described in [16]. Initially, the frozen fungal culture liquid was thawed in a water bath at 22°C for 1 h and insoluble material was removed by consecutive centrifugation at 4400×g for 25 min and gravity filtration of the supernatant through grade 597-1/2 quantitative folded filter paper with 4-7 µm pore size and a diameter of 320 mm (Whatman GmbH, Germany). The pH of the filtered solution was adjusted to 5 by addition of 1 M sodium acetate solution. To the supernatant was added Berol 532 detergent (AkzoNobel N.V., The Netherlands) to a final concentration of 2% (w/v). The solution was mixed by gentle shaking and allowed to separate in a separation funnel. The detergent phase was isolated followed by the addition of 20 ml of 50 mM sodium acetate buffer (pH 5.15) containing 40 mM EDTA were and 0.18 ml 2-methylpropan-1-ol. The mixture was mixed gently and the phases were allowed to separate in a separation funnel. The lower phase was purified further by preparative reversed-phase chromatography using a Vydac C4 (1 × 20 cm) column and a gradient elution from an aqueous solution containing 0.1% trifluoroacetic acid to 100% acetonitrile containing 0.1% trifluoroacetic acid. HFBI variant proteins eluting as fractions with a high absorption at λ=230 nm were pooled and lyophilized.

### Size exclusion chromatography (SEC)

HFBI variants dissolved in 100 µL buffer were injected at varying concentrations into a Superdex 75 column (Amersham, Sweden) and eluted with 10 mM Na-acetate buffer at pH 5.5 containing 0.2 M NaCl using an Äkta Explorer (Amersham, Sweden) HPLC system. Protein elution was detected with at λ = 230 nm. Reference proteins were injected to correlate the elution time with molecular weight. The references used were vitamin B-12 (1.4 kDa, Sigma), aprotinin (6.5 kDa, Sigma), ribonuclease A (13.7 kDa, Amersham Pharmacia Biotech, Sweden), and ovalbumin (43.0 kDa, Amersham Pharmacia Biotech, Sweden).

### Measurement of Isoelectric Point

The samples were prepared by dissolving wild type and variants of HFBI in ddH₂O at a concentration of 0.1 mg/mL, and the pH was adjusted using 100 mM and 1.0 M HCI and 10 and 100 mM NaOH. The zeta-potential was measured using a Zetasizer Nano-ZS instrument (Malvern, UK) and a sample volume of 1.0 ml.

### Drop flattening measurements

The time required for plateau formation at a hydrophobin containing drop was measured with an optical contact angle and surface tension meter (CAM 200, KSV NIMA, Finland). This was done by dissolving the studied protein dissolved in ddH₂O and pipetting a 50 µL drop of this solution onto a Parafilm sheet. Pictures were taken with 60 s interval in order to detect initial drop flattening moment, at which the drop started to form a plateau. The reported drop flattening times were determined as the average of three measurements.

### Formation and static analysis of HFBI monolayers

Formation of HFBI variant monolayers was analysed in a humidified atmosphere using a Langmuir trough surface pressure sensor and pre-soaked 20.6 mm perimeter Wilhelmy paper plates (KSV NIMA, Finland). Surface pressure was monitored by probing 20 ml of 5 mM Na-acetate (pH 5.5) in a glass beaker that has been equilibrated to room temperature. The sample protein was dissolved at a concentration of 0.85-1.0 µM by short magnetic stirring (1.5-2 min) and the change of surface pressure was monitored until an equilibrium was reached (typically after 30 min to 1 h).

### HFBI layer compression assay using the Langmuir trough

Monolayers of HFBI variants were assembled and its structural properties were measured using a KSV Minimicro trough (KSV NIMA, Finland). Protein layers were prepared at the air-water interface by injecting 20 µg dissolved protein into 55 ml of 5 mM Na-acetate buffer (pH 5.5) at 21°C. This solution was left for 45 min in order to allow the hydrophobin proteins to reach an equilibrium between an immersed state and the air-water interface assembly. Compression/expansion cycle isotherms were recorded while moving the barriers at a constant rate of 2 mm/min and switching to expansion at 35 mN/m surface pressure.

### Interfacial Rheology

The interfacial shear rheological properties of wild type HFBI and HFBI variants at the air-water interface were measured at RT using a DHR-2 rheometer (TA Instruments, U.K.) equipped with a Pt-Ir du Noüy ring (13 mm diameter, Krüss, Germany, flamed prior to use) in oscillatory mode. The concentrations of stock solutions of HFBI and HFBI variants were determined by UPLC. A 0.3 µM solution (≈ 2.3µg/ml = 0.00023% (w/v)) of HFBI (or variant) in Milli-Q water was prepared to a 50 ml volumetric flask and let to temper to RT (22°C) for 1 h prior to experiments. The solution was placed in a glass dish of 60 mm diameter and the du Noüy ring was placed onto the surface according to the manufacturer's instructions. The interfacial shear elastic (storage) modulus, G', and viscous (loss) modulus, G" were monitored at constant frequency of 0.1 Hz and constant strain of 0.1%, during film formation for at least 2 h to reach the equilibrium. The chosen frequency and strain were measured to be in the linear viscoelastic region. Each measurement was replicated at least twice.

### Atomic Force Microscopy (AFM)

A NanoScopeV Multimode8 AFM (E scanner, Bruker) and NCS15/AIBS cantilevers (µMasch, USA) were used in all measurements. All images were recorded in tapping mode in air with scan rates 0.7-1 Hz (free amplitude about 0.68V, damping ratio 0.7-0.8). Images were only flattened to remove possible tilt in the image data (NanoScope Analysis), and no further processing was done. A scanning probe image processor (SPIP, Image Metrology, Denmark) was used for image analysis. The topography and phase contrast images were captured simultaneously. The phase contrast image shows the phase difference between the oscillations of the cantilever driving piezo and the detected oscillations. It is thought that image contrast is derived from surface properties such as stiffness and viscoelasticity (hard tapping) or hydrophilicity/hydrophobicity (light tapping), but it also shows enhanced edge structures.

### QCM-D measurements

Hydrophobic protein adhesion and adsorption of sample proteins to HFBI variant layers at various pH's were monitored by QCM-D through measurement of frequency and dissipation (D4-QCM system, Biolin Scientific AB, Sweden). QCM sensors with a hydrophobic sensor surface were prepared by first cleaning gold-coated QCM sensor disks (model QSX301, Biolin Scientific AB, Sweden) in an UV/ozone chamber for 10 min and exposing them for another 10 min to a 5:1:1 mixture of ddH₂O, H₂O₂ and an aqueous ammonia solution (25% w/w) at ∼75°C. This was followed by thorough rinsing with ddH₂O, drying under a stream of N₂ and a 2-min incubation in ethanol (94% w/v). The pre-wetted sensors disks were immersed in a 50 mM 1-hexanethiol solution in ethanol and left to react at room temperature overnight. As a final step, unreacted 1-hexanethiol was washed off with ethanol and ddH₂O and the sensor was dried under a flow of N₂. The QCM-D measurements were performed at 23°C and buffer/sample injection was performed at a rate of 0.1 ml/min. All buffers were degassed by vacuum filtration before use. HFBI variant adsorption was carried out by injection of 300 µl protein sample solution containing HFBI at a concentration of 0.1 mg/ml and unbound protein was removed from the sensor surface by rinsing at pH 5.5 using 10 mM Na-acetate buffer.

### Results

The presented experiments were intended to improve the understanding of the molecular basis for hydrophobin monolayer formation and for the adsorption of secondary proteins to hydrophobin monolayers. In particular this study addresses the roles of charged hydrophobin residues that are involved in this process. These can be classified in two categories, e.g. the solvent exposed residues D30, K32, D40, D43, R45 and K50 (Figure 1A) and the residues D30 which K32 that are inaccessible to the solvent due to their location inside the protein layer in interaction (Figure 10). The charged residues proximal to the hydrophobic patch (D30 and K32) were also identified in computational studies as potentially important for intermolecular interactions [21] (Figure 1B).

### Production of HFBI variants with substituted charged amino acids

In order to study the roles of charged HFBI side chains, *Trichoderma reesei* strains were created with modified chromosomal DNA that contained added HFBI genes in which charged residues were substituted with sterically similar but uncharged homologues. Prior to these gene insertions, the gene *hfb2* encoding for the naturally secreted hydrophobin HFBII had to be disrupted because it would interfere with the HFBI variant purification. This was performed by insertion of a hygromycin resistance cassette in the *hfb2* gene of the *T. reesei* protein production strain M219. The cassette was amplified from plasmid pTNS27 essentially as described in [17]. Knock-out mutants were isolated by initial plating on hygromycin-containing top agar [150 µg/ml] and consecutive transfer to agar plates containing 0.1% (w/v) Triton X-100 and hygromycin [125 µg/ml]. The *hfb2* gene disruption was confirmed by PCR (Phire Plant Direct PCR kit (Thermo, Finnzymes F-130)) using two probes that were complementary to the *hph* resistance marker (SEQ ID NO:3; hph_sekv 1: 5'-ATTCTTCGCCCTCCGAGAGC-3') and the *hfb2* region located upstream of the deletion cassette insertion (SEQ ID NO:4; Mil73_fw: 5'-GAATCATGCTGGAGTGAAGG-3'). In addition, the absence of HFBII in the culture supernatant was confirmed by Western analysis (4-20% acrylamide Criterion gradient gel, Bio-Rad with HFBII-specific polyclonal antibodies Data not shown). The *T. reesei hfb2::hph* strain was deposited at the VTT culture collection under the ID D-121447.

The strain D-121447 was used for cloning of the HFBI variants using homologous recombination. This technique replaced a part of the highly expressed *cbhI* gene with an insertion cassette containing the acetamide resistance gene *amdS* and a HFBI variant gene downstream of the *cbhI* promotor. Four vectors containing this cassette were produced that differed in the encoded HFBI variant (E.g.: pMil003: - D40Q/D43N/R45Q/K50Q, pMil004: -D40Q/D43N, pMil005: -R45Q/K50Q and pMil006: -D30N/K32Q). The homologous recombination and clone selection was performed as described above with the exception that acetamide [10 mM] was used as a selective antibiotic. Acetamide-resistant clones were analysed for HFBI variant gene insertion by sequencing using primers that bind to the CBHI promoter (SEQ ID NO:5; T010_Cbh1 prom 5' sekv: 5'-CAACTCAGATCCTCCAGGAGAC-3') and CBHI terminator (SEQ ID NO:6; T043_Cbh1_term_R sekv: 5'-TCATGATACGGGCTCACCAAG-3'). Secretion of HFBI variants was confirmed by subjecting culture supernatants to Western blot analysis using polyclonal HFBI-specific antibodies. The obtained HFBI-variant secreting *Trichoderma reesei* strains were deposited at the VTT Culture collections with the IDs D-121448 (-D40Q/D43N/R45Q/K50Q), D-121449 (-D40Q/D43N), D-121450 (-R45Q/K50Q) and D-121451(-D30N/K32Q).

*T. reesei* variants were secreting the HFBI variants to the supernatant (Figure 8) at high concentrations that could be purified by the ATPS method yielding 100-500 mg per litre of shake flask culture (D30N/K32Q: 244.7 mg/l, D40Q/D43N: 98.5 mg/l, R45Q/K50Q: 390 mg/l and D40Q/D43N/R45Q/K50Q: 504 mg/l). Zeta-potential measurements showed that the introduced amino acid substitutions resulted in changes of the isoelectric point as expected according to the type of the modified acid (Figure 2 and Table 1).

**Table 1. Experimentally determined molecular weight (MW) according to MALDI-TOF mass spectrometry, isoelectric point (IEP) determined from duplicate measurements with theoretical value based on amino acid sequence given in brackets. The hydrophobically adsorbed mass (HAM) was determined from the presented QCM experiments on hexanethiol coated surfaces. The molecular area (MA) and the surface tension of HFBI variant solutions (γ) were determined from interfacial HFBI protein layers assembled in a Langmuir trough.**

| | D40Q/D43N | R45Q/K50Q | D30N/K32Q | D40Q/D43N/R45Q/K50Q | wild type |
|---|---|---|---|---|---|
| MW [Da] | 7546 | 7506 | 7533 | 7517 | 7535 |
| IEP | 7.0 (8.2) | 3.6 (3.9) | 5.8 (5.7) | 6.2 (5.5) | 6.1 (5.7) |
| HAM [ng/cm²] | 289 ± 45 | 217 ± 35 | 247 ± 35 | 293 ± 56 | 223 ± 98 |
| MA [Å²] | 323 ± 34 | 324 ± 55 | 322 ± 52 | 343 ± 38 | 323 ± 51 |
| γ[mN/m] | 31.3 ± 7.5 | 36.0 ± 0.6 | 36.5 ± 0.0 | 36.6 ± 0.3 | 35.5 ± 1.6 |

### Size-exclusion chromatography (SEC)

The concentration-dependent aggregation of the studied HFBI variants was studied by dissolving the protein at concentrations ranging between 3 µM and 1 mM, separation over a SEC column and monitoring their elution. At concentrations below 30 µM the four HFBI variants and the wild type were all at around 5 kDa corresponding to the monomer (7.5 kDa). Larger aggregates with a molecular weight corresponding to hexamers were formed when the hydrophobin concentration was increased above 100 µM, (Figure 3). The transition between these two states appeared to resemble the wild type in the case of all tested variants.

### Assembly of hydrophobin variant layers at the air-water interface

The surface activity of hydrophobins can be easily observed when a drop of hydrophobin solution is left to evaporate and a plateau formation can be observed at the top facing part of the drop due to the concentration-dependent formation of a hydrophobin monolayer at the air-water interface. In order to test the ability of the HFBI variants to participate in this process, droplets of hydrophobin solution [0.1 mg/ml in 5.0 mM Na acetate buffer (pH 5.5)] were left to evaporate and the time necessary for plateau formation determined using the CAM apparatus. For all four variants, the time until drop flattening was 30-70% longer than the incubation period required by the wild type (19 ± 3.5 min).

The molecular area occupied in an HFBI variant layer at the air-water interface was determined with the Langmuir-Blodgett apparatus by measurement of the 2^{nd} compression isotherm. Here, no clear difference in molecular area could be seen to the wild type HFBI (3.23 ± 0.51 nm²) (see Table 1).

In an additional Langmuir-Blodgett apparatus experiment, the surface tension reduction upon HFBI variant layer formation at the air-water interface was measured. No significant difference could be seen in surface tension reduction between the variants (D40Q/D43N: 31.3 ± 7.5 mN/m, R45Q/K50Q: 36.0 ± 0.6 mN/m, D40Q/D43N/R45Q/K50Q: 36.6 ± 0.3 mN/m, D30N/K32Q: 36.5 ± 0.0 mN/m) and the wild type (35.5 ± 1.6 mN/m) (see Table 1).

### Interfacial rheology of hydrophobin variant layers

The adsorption of wild type HFBI and HFBI variants to the air-water interface was monitored and viscoelastic properties determined by interfacial shear rheology measurements. The storage modulus (elastic, G') and loss modulus (viscous, G") of the interfacial hydrophobin layers are shown in Figures 4A and 4B, respectively. At equilibrium the shear storage modulus, G', for wild type HFBI reached a value of 1.04 ± 0.01 N/m, which is comparable to previously reported values for HFBI (0.7 N/m in [15]). The corresponding values for the HFBI variants D30N/K32Q, D40Q/D43N, R45Q/K50Q and D40Q/D43N/R45Q/K50Q are 0.85 ± 0.10 N/m, 0.62 ± 0.01 N/m, 1.44 ± 0.03 N/m and 1.09 ± 0.01 N/m, respectively. The G' values are in the similar range or less than for wild type HFBI, except the high value for R45Q/K50Q variant that showed ∼40% increase in the elastic modulus. The equilibrium shear loss modulus values, G" (Figure 4B) for all hydrophobins were less than 0.06 N/m. Tanδ values at equilibrium were very low, less than 0.1, for all hydrophobins showing dominance of the G' values in all cases.

### AFM imaging of hydrophobin variant layers as Langmuir-Blodgett films

AFM images of Langmuir-Blodgett films (LB-films) of all HFBI-variants showed regular ordered raft-like structures (Figure 5A-D) of different variant-specific dimensions (e.g.: D30N/K32Q: ∼150×150 nm, D40Q/D43N: ∼120×120 nm, D40Q/D43N/R45Q/K50Q: ∼350×350 nm and R45Q/K50Q: ∼800×800 nm). It is possible that at the air-water interface all protein films consisted of larger crystalline domains, which broke down to smaller rafts during film transfer onto mica. The crystallinity of the protein films was confirmed by Fourier transformation of the phase contrast image data. The oblique structures of all HFBI-variants possessed unit cell vector dimensions of 5.4 ± 0.9 nm for a and b, and 120 ± 12° for Y. The deviation of the lattice constants from pure hexagonal packing (a=B and Y=120°) and differences between different samples can be explained by scanner hysteresis, creep and drift in the AFM. These phenomena become more dominant when imaging in relatively slow scan speeds.

### QCM measurements of pH-dependent binding of glucose oxidase to HFBI variants

The binding of proteins to hydrophobin layers was investigated by QCM in order to determine whether the regular charge pattern of a HFBI monolayer (Figure 1B)_is recognized by other proteins. This was done by adding secreted carbohydrate-active *T. reesei* enzymes and, as a reference, *Aspergillus niger* glucose oxidase to hydrophobically adsorbed layers of wild type hydrophobin and the four variants as outlined in Figure 6A and B.

The adsorption to a hydrophobic hexanethiol coated surface was found to be very similar among the tested HFBI variants and the HFBI wildtype (Table 1). With all variants, maximum GOx adsorption was measured at either pH 4.7 or 5.2 (Figure 7A) which was slightly above its pI of 4.2. Interestingly, only the basic R45Q/K50Q variant bound GOx at pH 6-9. Dominant electrostatic repulsion at pH ≠ pI and non-electrostatic attractive forces at pH = pI can explain the observed pH-dependent GOx binding. No significant binding of the *T*. *reesei* enzymes xylanase I (pI 9), xylanase II (pI 5.5), cellobiohydrolase I (pI 3.7-4.2) and endoglucanase II (pI 5.2) could be measured for HFBI wild type layers and layers of variants D30N/K32Q, R45Q/K50Q and D40Q/D43N/R45Q/K50Q at the probed pH range (Fig. 7B-E). Also in the case of HFBI variant D40Q/D43N, no binding of xylanase (Fig. 7B) an endoglucanase II (Fig. 7E) could be seen but minor amounts (55-100 ng/cm²) of xylanase II were bound in a pH-independent fashion (Fig. 7C). Clear pH-dependent adsoption of cellobiohydrolase I on the D40Q/D43N coated substrate could be seen between pH 3 and 7 with about 600 ng/cm² maximum adsorption at around pH 5.

### Discussion

Previously published experiments on HFBI wild type layers have shown that the adsorption of some proteins (e.g. *A. niger* glucose oxidase, BSA, IgG and avidin) is highly pH-dependent [9] and indicated that the HFBI charges could play an important role in the adsorption process. In order to increase the understanding of the role of HFBI charges for HFBI-protein interaction, the mutation experiments that are presented in this study were performed. Here, we explore the role of charged HFBI side chains during the HFBI monolayer assembly and their ability to function as possible interaction partners for other proteins during charge-dependent adsorption to the HFBI monolayer. For this purpose charge variants were created where either two or all similarly charged surface-exposed residues were exchanged against sterically related non-charged homologues (e.g. D40Q/D43N, R45Q/K50Q and D40Q/D43N/R45Q/K50Q, respectively). In addition, the role of the counter charge pair D30-K32 close to the hydrophobic patch (Fig. 1) was investigated in a fourth mutant (D30N/K32Q). The yields of purified protein obtained from the culture supernatants by ATPS showed that the protein concentration in case of the variants were 244.7 mg/l (D30N/K32Q), 390 mg/l (R45Q/K50Q) and 504 mg/l (D40Q/D43N/R45Q/K50Q). In case of the wild type HFBI, the highest protein yields so far have been obtained from the mycelium (about 100 mg/l) [18]. Thereby, the mentioned HFBI amino acid substitutions improved the yields of purified HFBI two-to fourfold. This improvement may become relevant for technical applications where HFBI variant purification from a supernatant is preferable over the preparation from the mycelium.

The mutations introduced into the HFBI sequence had significant effects on the protein isoelectric point (Fig. 2, Table 1) which were found in most cases to be close to the theoretical values that were calculated from the corresponding amino acid sequences (Table 1). Some deviations to more acidic values were apparent such as in the case of variant D40Q/D43N (7.0 measured vs. 8.2 calculated). These deviations could be explained by the orientation of the residue R45 away from the surface which might prevent the guanidinium group from interaction with the solvent and thereby lead to a lower pI than what is expected from the amino acid sequence.

The concentration-dependent oligomerisation of HFBI variants studied by SEC was found to closely resemble the wild type behavior. Interestingly, some of these HFBI mutations had a clear effect on the oligomerisation tendency when the hydrophobin was linked to an N-terminal GFP domain. In the GFP-HFBI protein mutants D40Q/D43N and D40Q/D43N/R45Q/K50Q a shift to smaller aggregates was seen whereas the D30N/K32Q substitutions induced formation of oligomers that were larger than the predominant wild type aggregates [19]. A possible explanation for this differential behavior could be a re-orientation of the fusion partner that is induced by the mutations and effects GFP-HFBI aggregation but cannot be observed with the isolated HFBI variants.

The importance of the charged HFBI residues for monolayer assembly at the air-water interface was investigated using interfacial shear rheology, a Langmuir trough and AFM. When hydrophobin layers at the air-water interface were tested for surface tension reduction and mean molecular area no significant difference could be seen between the wild type and the four variants. These results suggest that the molecular dimensions of the HFBI variants in the interfacial monolayer remained largely unaffected by the performed amino acid substitutions which correspond well to the subtle nature of these structural alterations.

Interfacial shear rheology measurements were carried out for wild type HFBI and HFBI variants in order to see the effect of mutations on viscoelastic properties of protein layers at the air-water interface. Response of the interfacial layer is sensitive to protein-protein interactions, and the experiments were hence expected to show differences between the HFBI variants. The interfacial shear moduli G' and G" (Figure 4A), and consequently the tanδ values show that all studied hydrophobins form strong elastic layers. The G' values are remarkably high for all: HFBI 1.04 ± 0.01 N/m, D30N/K32Q 0.85 ± 0.10 N/m, D40Q/D43N 0.62 ± 0.01 N/m, R45Q/K50Q 1.44 ± 0.03 N/m and D40Q/D43N/R45Q/K50Q 1.09 ± 0.01 N/m. In comparison with another set of widely studied surface active proteins, milk proteins possess only a fraction of the strength determined by interfacial shear rheology for hydrophobins (1 µM β-lactoblobulin less than 5 mN/m, 1 µM β-casein close to zero; [20]). The present inventors want to point out especially the storage modulus value for HFBI-R45Q/K50Q, 1.44 N/m, which to the present inventor's knowledge is the highest interfacial shear rheology value reported for an interfacial protein layer.

The loss modulus, G", of wild-type HFBI showed an interesting behavior during adsorption (Figure 4B). The adsorption process for all hydrophobins showed first increase in loss modulus, passed through a maximum and then leveled out. This phenomenon may originate from rearrangements taking place at the interface before the protein layer reached equilibrium. The adsorption process seems to be different for the wild-type HFBI: the loss modulus undergoes through two maxima before reaching equilibrium. The mutations seem to affect the self-assembling characteristics as one way is lost in HFBI variants.

AFM imaging showed strong similarities between 2D-assemblies of the tested proteins and the highest mica coverage for the variant R45Q/K50Q at a raft size of ∼800×800 nm. This was area was significantly higher than the raft size measured for the other three variants (≤300×300 nm) and the HFBI wildtype, which was analyzed by AFM previously (∼500×500 nm) [16]. This finding correlates well with unpublished ellipsometry experiments, during which it was shown that the R45Q/K50Q formed a more dense layer at the air-water interface than the variants D30N/K32Q and D40Q/D43N/R45Q/K50Q as well as the HFBI wild type. The HFBI variant lattice constants measured from the AFM images equalled well to the lattice constants reported previously for LB-films of wild type HFBI ([16], [8]).

In another set of experiments, the idea of charge-dependent recognition of HFBI layers by secreted fungal enzymes was tested. Here, the previously published pH-dependency of GOx binding to HFBI wild type layers [9] was complemented by repeating the GOx adsorption experiment with the four HFBI charge variants and additionally monitoring adsorption of four secreted *T. reesei* enzymes. QCM showed that the wild type and the tested variants all formed similarly thick layers with a mass density of around 220 ng/cm² (see Table 1). These observations suggest that the HFBI layer structure was largely unaffected by the introduced mutations but the layer assembly kinetics were sensitive to the charge substitutions that were introduced into the HFBI sequence. Interestingly, none of the tested amino acid substitutions changed the optimal GOx-binding pH (∼4.5). However, the amount of GOx bound at this condition appeared to be HFBI-charge sensitive and an additional interaction was found for the variant D40Q/D43N at neutral pH. This indicated that binding to the HFBI wild type layer at neutral pH is hindered by the negatively charged aspartates. When *Trichoderma* enzymes were screened for binding to the HFBI layer, the highest binding was measured at slightly acidic pH for CBHI and some binding with XYLII to the HFBI variant D40Q/D43N while lower adhesion was measured for these enzymes when they were added to HFBI wild type layers. The observed strongly pH-dependent charge interaction support the idea that HFBI layers can be recognized by certain proteins and some regulation by charged HFBI residues could be involved as these were seen to strongly interfere with the binding of other proteins to the HFBI layer. The increased binding of variant D40Q/D43N to certain proteins could be taken as a rationale to explain the differential mutant-specific aggregation properties that were reported here for isolated HFBI variants and previously for GFP-HFBI fusion proteins [19]. E.g., it was previously reported that the D40Q/D43N substitution decreased the predominant aggregation state of the GFP-HFBI protein from a tetramer to a monomer. The isolated HFBI protein, however, appeared to be unaffected in its aggregation properties by the D40Q/D43N substitution as shown in the present study. This reason for this difference could be a strongly adsorbed GFP domain at the HFBI surface in GFP-HFBI and sterically hinders the assembly of fusion oligomers in solution.

### References

1. Cox AR, Aldred DL, Russell AB. Exceptional stability of food foams using class II hydrophobin HFBII. Food Hydrocolloid. 2009 Mar;23(2):366-76.
2. Latge JP, Bouziane H, Diaquin M. Ultrastructure and Composition of the Conidial Wall of Cladosporium-Cladosporioides. Can J Microbiol. 1988 Dec;34(12):1325-9.
3. Boualem K, Wache Y, Garmyn D, Karbowiak T, Durand A, Gervais P, et al. Cloning and expression of genes involved in conidiation and surface properties of Penicillium camemberti grown in liquid and solid cultures. Res Microbiol. 2008 Mar;159(2):110-7.
4. Askolin S, Penttila M, Wosten HAB, Nakari-Setala T. The Trichoderma reesei hydrophobin genes hfb1 and hfb2 have diverse functions in fungal development. Fems Microbiol Lett. 2005 Dec 15;253(2):281-8.
5. Wosten HAB, van Wetter MA, Lugones LG, van der Mei HC, Busscher HJ, Wessels JGH. How a fungus escapes the water to grow into the air. Curr Biol. 1999 Jan 28;9(2):85-8.
6. Wosten HAB, Willey JM. Surface-active proteins enable microbial aerial hyphae to grow into the air. Microbiol-Uk. 2000 Apr;146:767-73.
7. Linder MB. Hydrophobins: Proteins that self assemble at interfaces. Curr Opin Colloid In. 2009 Oct;14(5):356-63.
8. Szilvay GR, Paananen A, Laurikainen K, Vuorimaa E, Lemmetyinen H, Peltonen J, et al. Self-assembled hydrophobin protein films at the air-water interface: Structural analysis and molecular engineering. Biochemistry-Us. 2007 Mar 6;46(9):2345-54.
9. Wang ZF, Lienemann M, Qiau M, Linder MB. Mechanisms of Protein Adhesion on Surface Films of Hydrophobin. Langmuir. 2010 Jun 1;26(11):8491-6.
10. Thompson JD, Higgins DG, Gibson TJ. Clustal-W - Improving the Sensitivity of Progressive Multiple Sequence Alignment through Sequence Weighting, Position-Specific Gap Penalties and Weight Matrix Choice. Nucleic Acids Res. 1994 Nov 11;22(22):4673-80.
11. Linder MB, Szilvay GR, Nakari-Setala T, Penttila ME. Hydrophobins: the protein-amphiphiles of filamentous fungi. Fems Microbiol Rev. 2005 Nov;29(5):877-96.
12. Kisko K, Szilvay GR, Vuorimaa E, Lemmetyinen H, Linder MB, Torkkeli M, et al. Self-assembled films of hydrophobin protein HFBIII from Trichoderma reesei. J Appl Crystallogr. 2007 Apr;40:S355-S60.
13. Gonnet GH, Cohen MA, Benner SA. Exhaustive Matching of the Entire Protein-Sequence Database. Science. 1992 Jun 5;256(5062):1443-5.
14. Hakanpaa J, Paananen A, Askolin S, Nakari-Setala T, Parkkinen T, Penttila M, et al. Atomic resolution structure of the HFBII hydrophobin, a self-assembling amphiphile. J Biol Chem. 2004 Jan 2;279(1):534-9.
15. Cox AR, Cagnol F, Russell AB, Izzard MJ. Surface properties of class II hydrophobins from Trichoderma reesei and influence on bubble stability. Langmuir. 2007 Jul 17;23(15):7995-8002.
16. Paananen A, Vuorimaa E, Torkkeli M, Penttila M, Kauranen M, Ikkala O, et al. Structural hierarchy in molecular films of two class II hydrophobins. Biochemistry-Us. 2003 May 13;42(18):5253-8.
17. Penttila M, Nevalainen H, Ratto M, Salminen E, Knowles J. A Versatile Transformation System for the Cellulolytic Filamentous Fungus Trichoderma-Reesei. Gene. 1987;61(2):155-64.
18. Bailey MJ, Askolin S, Horhammer N, Tenkanen M, Linder M, Penttila M, et al. Process technological effects of deletion and amplification of hydrophobins I and II in transformants of Trichoderma reesei. Appl Microbiol Biot. 2002 May;58(6):721-7.
19. Lienemann M, Gandier J-A, Joensuu JJ, Iwanaga A, Takatsuji Y, Haruyama T, et al. Structure-function relationships in hydrophobins: Probing the role of charged side chains. Appl Environ Microb. 2013;79(18):5533-8.
20. Mackie AR, Gunning AP, Ridout MJ, Wilde PJ, Morris VJ. Orogenic displacement in mixed beta-lactoglobulin/beta-casein films at the air/water interface. Langmuir. 2001 Oct 16;17(21):6593-8.
21. Magarkar, A., Mele, N., Abdel-Rahman, N., Unger, V. M., Butcher, S., Torkkeli, M., Serimaa, R., Paananen, A., Linder, M., and Bunker, A. Hydrophobin film structure for HFBI and HFBII and mechanism for accelerated film formation. PLoS Comput. Biol., 10(7), e1003745.
22. Kloek, W., van Vliet, T., & Meinders, M., Effect of bulk and interfacial rheological properties on bubble dissolution. Journal of Colloid and Interface Science 2001, 237; 158-166.
23. Yamasaki, R., Takatsuji, Y., Lienemann, M., Asakawa, H., Fukuma, T., Linder, M., Heruyama, T. Electrochemical properties of honeycomb-like structured HFBI self-organized membranes on HOPG electrodes. Colloids. Surf. B: Biointerfaces 2014, http://dx.doi.org/10.1016/j.colfurfb.2014.10.018.

## Claims

1. A composition comprising a hydrophobin variant of class II hydrophobin **characterized in that** the variant comprises an amino acid sequence having a substitution of charged amino acid residues against sterically related non-charged homologues in amino acid sequence of the mature hydrophobin HFBI of SEQ ID NO:2, wherein said substitutions are R45Q and K50Q, wherein the positions of the amino acid substitutions are numbered in reference to the positions in SEQ ID NO:2 and wherein the variant has improved formation of an elastic protein film compared to the wild type mature hydrophobin HFBI of sequence SEQ ID NO:2.

2. The composition according to claim 1 **characterized in that** the variant comprises as a fusion partner a fusion protein of equal or smaller size than said hydrophobin variant.

3. The composition according to any of the claims 1 or 2 **characterized in that** the variant is *Trichoderma reesei* hydrophobin HFBI variant.

4. The composition according to any one of claims 1 to 3, **characterized in that** the variant is SEQ ID NO:10.

5. The composition according to claim 4 **characterized in that** said variant has improved secretion to the *Trichoderma reesei* culture supernatant when compared to the wild type mature hydrophobin HFBI of sequence SEQ ID NO:2.

6. A method for increasing product stability in a product containing air-water or oil-water interface, **characterized in that** the method comprises the step of using the hydrophobin composition of any one of claims 1 to 5.

7. The method according to claim 6, **characterized in that** product stability is increased by forming a strong and stable foam or emulsion, a strong elastic film, or providing improved visco-elastic properties of interfacial films.

8. The method according to claim 6 or 7, **characterized in that** the product containing air-water or oil-water interface is selected from the group consisting of food products, personal care products, paints, pharmaceuticals, and drugs.

9. Use of the hydrophobin composition according to any one of claims 1 to 5 as a stabilizer, preferably as a stabilizer in foams or emulsions.

10. Use of the hydrophobin composition according to any one of claims 1 to 5 in personal care products or paints.

## Patentansprüche

1. Zusammensetzung, die eine Hydrophobin-Variante von Klasse-II-Hydrophobin umfasst, **dadurch gekennzeichnet, dass** die Variante eine Aminosäuresequenz umfasst, die eine Substitution von geladenen Aminosäureresten gegen sterisch verwandte nichtgeladene Aminosäuresequenz-Homologe des reifen Hydrophobins HFBI von SEQ-ID NO:2 aufweist, wobei die Substitutionen R45Q und K50Q sind, wobei die Positionen der Aminosäuresubstitutionen unter Verweis auf die Positionen in SEQ ID NO:2 nummeriert sind und wobei die Variante eine verbesserte Ausbildung eines elastischen Proteinfilms im Vergleich zum wildtypischen reifen Hydrophobin HFBI der Sequenz SEQ ID NO:2 aufweist.

2. Zusammensetzung nach Anspruch 1, **dadurch gekennzeichnet, dass** die Variante als Fusionspartner ein Fusionsprotein von gleicher oder geringerer Größe als die Hydrophobinvariante umfasst.

3. Zusammensetzung nach einem der Ansprüche 1 oder 2, **dadurch gekennzeichnet, dass** die Variante die Hydrophobin-HFBI-Variante *Trichoderma reesei* ist.

4. Zusammensetzung nach einem der Ansprüche 1 bis 3, **dadurch gekennzeichnet, dass** die Variante SEQ ID NO:10 ist.

5. Zusammensetzung nach Anspruch 4, **dadurch gekennzeichnet, dass** die Variante eine verbesserte Sekretion in den *Trichoderma-reesei-*Kulturüberstand im Vergleich zum wildtypischen reifen Hydrophobin HFBI der Sequenz SEQ ID NO:2 aufweist.

6. Verfahren zur Erhöhung der Produktstabilität in einem Produkt, das eine Luft/Wasser- oder Öl/Wasser-Grenzfläche enthält, **dadurch gekennzeichnet, dass** das Verfahren den Schritt der Verwendung der Hydrophobin-Zusammensetzung nach einem der Ansprüche 1 bis 5 umfasst.

7. Verfahren nach Anspruch 6, **dadurch gekennzeichnet, dass** die Produktstabilität durch die Ausbildung eines festen und stabilen Schaums oder einer festen und stabilen Emulsion, eines festen elastischen Films oder durch die Bereitstellung von verbesserten viskoelastischen Eigenschaften von Grenzflächenfilmen erhöht wird.

8. Verfahren nach Anspruch 6 oder 7, **dadurch gekennzeichnet, dass** das eine Luft/Wasser- oder Öl/Wasser-Grenzfläche enthaltende Produkt ausgewählt ist aus der Gruppe bestehend aus Lebensmittelprodukten, persönlichen Pflegeprodukten, Anstrichstoffen, Pharmaka und Arzneimitteln.

9. Verwendung der Hydrophobin-Zusammensetzung nach einem der Ansprüche 1 bis 5 als Stabilisator, vorzugsweise als Stabilisator in Schäumen oder Emulsionen.

10. Verwendung der Hydrophobin-Zusammensetzung nach einem der Ansprüche 1 bis 5 in persönlichen Pflegeprodukten oder Anstrichstoffen.

## Revendications

1. Composition comprenant une variante d'hydrophobine d'une hydrophobine de classe II, **caractérisée en ce que** la variante comprend une séquence d'acide aminé ayant une substitution de résidus d'acide aminé chargés contre des homologues non chargés stériquement similaires à la séquence d'acide aminé de l'hydrophobine mature HFBI de SEQ ID NO:2, lesdites substitutions étant R45Q et K50Q, les positions desdites substitutions d'acide aminé étant numérotées en référence aux positions en SEQ ID NO:2 et ladite variante ayant une formation améliorée d'un film protéinique élastique en comparaison avec l'hydrophobine mature HFBI de type sauvage de la séquence SEQ ID NO:2.

2. Composition selon la revendication 1, **caractérisée en ce que** la variante comprend comme partenaire de fusion une protéine de fusion dont la taille est égale ou inférieure à celle de ladite variante d'hydrophobine.

3. Composition selon l'une des revendications 1 ou 2, **caractérisée en ce que** la variante est la variante d'hydrophobine HFBI *Trichoderma reesei.*

4. Composition selon l'une des revendications 1 à 3, **caractérisée en ce que** la variante est SEQ ID NO:10.

5. Composition selon la revendication 4, **caractérisée en ce que** ladite variante a une sécrétion améliorée dans le surnageant de culture de *Trichoderma reesei* en comparaison avec l'hydrophobine mature HFBI de type sauvage de la séquence SEQ ID NO:2.

6. Procédé d'augmentation de la stabilité de produit dans un produit contenant une interface air-eau ou huile-eau, **caractérisé en ce que** le procédé comprend l'étape d'utilisation de la composition d'hydrophobine selon l'une des revendications 1 à 5.

7. Procédé selon la revendication 6, **caractérisé en ce que** la stabilité de produit est augmentée par la formation d'une mousse ou émulsion ferme et stable, d'un film élastique ferme ou par la fourniture de propriétés viscoélastiques améliorées des films interfaciaux.

8. Procédé selon la revendication 6 ou 7, **caractérisé en ce que** le produit contenant une interface air-eau ou huile-eau est choisi dans le groupe constitué par les produits alimentaires, les produits de soins personnels, les peintures, les pharmaceutiques et les médicaments.

9. Utilisation de la composition d'hydrophobine selon l'une des revendications 1 à 5 en tant que stabilisateur, préférablement en tant que stabilisateur dans les mousses ou émulsions.

10. Utilisation de la composition d'hydrophobine selon l'une des revendications 1 à 5 dans les produits de soins personnels ou les peintures.


### SEQUENCE LISTING

<110> Teknologian tutkimuskeskus VTT
<120> A Method for Increasing Stability
<130> B3655PFI
<160> 10
<170> PatentIn version 3.5
<210> 1
   <211> 97
   <212> PRT
   <213> Trichoderma reesei
<220>
   <221> SIGNAL
   <222> (1)..(16)
<220>
   <221> PROPEP
   <222> (17)..(22)
<400> 1
   Met Lys Phe Phe Ala Ile Ala Ala Leu Phe Ala Ala Ala Ala Val Ala
   1               5                   10                  15
   Gln Pro Leu Glu Asp Arg Ser Asn Gly Asn Gly Asn Val Cys Pro Pro
               20                  25                  30
   Gly Leu Phe Ser Asn Pro Gln Cys Cys Ala Thr Gln Val Leu Gly Leu
           35                  40                  45
   Ile Gly Leu Asp Cys Lys Val Pro Ser Gln Asn Val Tyr Asp Gly Thr
       50                  55                  60
   Asp Phe Arg Asn Val Cys Ala Lys Thr Gly Ala Gln Pro Leu Cys Cys
   65                  70                  75                  80
   Val Ala Pro Val Ala Gly Gln Ala Leu Leu Cys Gln Thr Ala Val Gly
                   85                  90                  95
   Ala
<210> 2
   <211> 75
   <212> PRT
   <213> Trichoderma reesei
<400> 2
   Ser Asn Gly Asn Gly Asn Val Cys Pro Pro Gly Leu Phe Ser Asn Pro
   1               5                   10                  15
   Gln Cys Cys Ala Thr Gln Val Leu Gly Leu Ile Gly Leu Asp Cys Lys
               20                  25                  30
   Val Pro Ser Gln Asn Val Tyr Asp Gly Thr Asp Phe Arg Asn Val Cys
           35                  40                  45
   Ala Lys Thr Gly Ala Gln Pro Leu Cys Cys Val Ala Pro Val Ala Gly
       50                  55                  60
   Gln Ala Leu Leu Cys Gln Thr Ala Val Gly Ala
   65                  70                  75
<210> 3
   <211> 20
   <212> DNA
   <213> Artificial Sequence
<220>
   <223> probe
<400> 3
   attcttcgcc ctccgagagc 20
<210> 4
   <211> 20
   <212> DNA
   <213> Artificial Sequence
<220>
   <223> probe
<400> 4
   gaatcatgct ggagtgaagg 20
<210> 5
   <211> 22
   <212> DNA
   <213> Artificial Sequence
<220>
   <223> primer
<400> 5
   caactcagat cctccaggag ac 22
<210> 6
   <211> 21
   <212> DNA
   <213> Artificial Sequence
<220>
   <223> primer
<400> 6
   tcatgatacg ggctcaccaa g 21
<210> 7
   <211> 75
   <212> PRT
   <213> Trichoderma reesei
<400> 7
   Ser Asn Gly Asn Gly Asn Val Cys Pro Pro Gly Leu Phe Ser Asn Pro
   1               5                   10                  15
   Gln Cys Cys Ala Thr Gln Val Leu Gly Leu Ile Gly Leu Asn Cys Gln
               20                  25                  30
   Val Pro Ser Gln Asn Val Tyr Asp Gly Thr Asp Phe Arg Asn Val Cys
           35                  40                  45
   Ala Lys Thr Gly Ala Gln Pro Leu Cys Cys Val Ala Pro Val Ala Gly
       50                  55                  60
   Gln Ala Leu Leu Cys Gln Thr Ala Val Gly Ala
   65                  70                  75
<210> 8
   <211> 75
   <212> PRT
   <213> Trichoderma reesei
<400> 8
   Ser Asn Gly Asn Gly Asn Val Cys Pro Pro Gly Leu Phe Ser Asn Pro
   1               5                   10                  15
   Gln Cys Cys Ala Thr Gln Val Leu Gly Leu Ile Gly Leu Asp Cys Lys
               20                  25                  30
   Val Pro Ser Gln Asn Val Tyr Gln Gly Thr Asn Phe Arg Asn Val Cys
           35                  40                  45
   Ala Lys Thr Gly Ala Gln Pro Leu Cys Cys Val Ala Pro Val Ala Gly
       50                  55                  60
   Gln Ala Leu Leu Cys Gln Thr Ala Val Gly Ala
   65                  70                  75
<210> 9
   <211> 75
   <212> PRT
   <213> Trichoderma reesei
<400> 9
   Ser Asn Gly Asn Gly Asn Val Cys Pro Pro Gly Leu Phe Ser Asn Pro
   1               5                   10                  15
   Gln Cys Cys Ala Thr Gln Val Leu Gly Leu Ile Gly Leu Asp Cys Lys
               20                  25                  30
   Val Pro Ser Gln Asn Val Tyr Gln Gly Thr Asn Phe Gln Asn Val Cys
           35                  40                  45
   Ala Gln Thr Gly Ala Gln Pro Leu Cys Cys Val Ala Pro Val Ala Gly
       50                  55                  60
   Gln Ala Leu Leu Cys Gln Thr Ala Val Gly Ala
   65                  70                  75
<210> 10
   <211> 75
   <212> PRT
   <213> Trichoderma reesei
<400> 10
   Ser Asn Gly Asn Gly Asn Val Cys Pro Pro Gly Leu Phe Ser Asn Pro
   1               5                   10                  15
   Gln Cys Cys Ala Thr Gln Val Leu Gly Leu Ile Gly Leu Asp Cys Lys
               20                  25                  30
   Val Pro Ser Gln Asn Val Tyr Asp Gly Thr Asp Phe Gln Asn Val Cys
           35                  40                  45
   Ala Gln Thr Gly Ala Gln Pro Leu Cys Cys Val Ala Pro Val Ala Gly
       50                  55                  60
   Gln Ala Leu Leu Cys Gln Thr Ala Val Gly Ala
   65                  70                  75